# EUROPEAN PATENT SPECIFICATION

(11) **EP 2 094 674 B1**
(45) Date of publication and mention of the grant of the patent: **16.03.2011**
(21) Application number: 07825549.4
(22) Date of filing: 31.10.2007
(51) Int. Cl.: C07D 261/20, A61K 31/423, A61P 25/00

(54) **A SALT OF 3-BENZYL-2-METHYL-2,3,3A,4,5,6,7,7A-OCTAHYDROBENZO[D]ISOXAZOL-4-ONE**
SALZ VON 3-BENZYL-2-METHYL-2,3,3A,4,5,6,7,7A-OCTAHYDROBENZO[D]ISOXAZOL-4-ON
SEL DE 3-BENZYL-2-METHYL-2,3,3A,4,5,6,7,7A-OCTAHYDROBENZO[D]ISOXAZOL-4-ONE

(30) Priority: 02.11.2006 IT MI20062102
(43) Date of publication of application: 02.09.2009
(73) Proprietor: Abiogen Pharma S.p.A., 56014 Località Ospedaletto (PI) (IT)
(72) Inventor: NEGGIANI, Fabio, 56123 Pisa (IT); DINI, Laura, 56127 Pisa (IT)
(74) Representative: Cattaneo, Elisabetta
(86) International application number: PCT/IB2007/003291
(87) International publication number: WO 2008/053325

(56) References cited:
- WO-A-93/17004

## Description

The present invention relates to fumarate salt of the compound of formula: 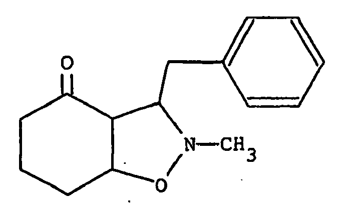 in all its stereochemical configurations, a process for its preparation and its use in treating mood disorders, disorders of anxiety, depression and convulsive conditions, in the improvement of learning ability, in the reversal of amnesia, in resolving the abstinence syndrome from drugs and drugs of abuse.

More specifically, the invention concerns fumarate salt ofrel-(3R,3aS,7aS)-3-benzyl-2-methyl-2,3,3a,4,5,6,7,7a-octahydrobenzo[d]isoxazol-4-one.

The compound rel-(3R,3aS,7aS)-3-benzyl-2-methyl-2,3,3a,4,5,6,7,7a-octahydrobenzo[d]isoxazol-4-one, also known as BTG 1640, is described in the International application with publication number WO93/17004 and belongs to a family of new psychoactive compounds.

Specifically, in the patent application with publication number WO93/17004 the tranquilizing activity of the whole compound family is proposed and particularly, the activity of the compound BTG 1640 and the effects thereof in learning ability and in the reversal of amnesia are tested.

According to such a document, the compound BTG 1640 is prepared as a yellow oil and, in tests demonstrating the proposed activity, it is used after dilution in PEG and distilled water.

In pharmaceutical practises, it is known that oil substances have a lot of drawbacks mostly linked to the difficult handling and formulation. The oils are in fact hard to weight, basically less stable to temperature variations, less soluble in ordinary solvents and therefore technically harder to dose for the preparation of pharmaceutical formulations.

It is generally advisable that the form of active ingredient is the crystalline solid one which normally shows better characteristics, specifically in terms of handling for activities of pharmaceutical formulation.

With regard to this, the patent application WO93/17004 cites the possibility of preparing the salt form of new psychoactive compounds of general Formula (I), by treating the free base of a compound of Formula (I) with the suitable free acid. Specifically, document WO93/17004 describes the hydrochloride of BTG 1640 obtained in the form of white crystalline powders.

By taking such a salt as a reference, and wishing therefore to avoid the use of free base BTG 1640 in oil form, in the step of drug formulation, the stability of such a hydrochloride salt as active principle was evaluated, in order to state the storage and preservation conditions of the drug. It is known that a pharmaceutical substance is deemed stable if it maintains the same initial characteristics, when subjected to different temperature and humidity conditions in time. As it will be demonstrated in the examples, the stability analysis showed that the hydrochloride salt of BTG 1640 is unstable at temperatures higher than 30°C, thereby refrigerator preservation at temperatures of 2-8°C becomes advisable, in precautional way, not only for the single salt as active substance, but also for the different pharmaceutical formulations, which are nowadays developed and based on such an active principle.

Such a refrigerator preservation condition, although necessary, results rather disadvantageous. As a matter of fact, the observation of such preservation precautions is scarcely accepted by the patient, who results evidently not accommodated in the daily practise and in his/her activities, by having to keep the drug in the refrigerator, out of necessity.

It is still felt the need of providing the compound 3-benzyl-2-methyl-2,3,3a,4,5,6,7,7a-octahydrobenzo[d]isoxazol-4-one in a form which does not require peculiar preservation conditions.

An object of the present invention is therefore to provide a form of 3-benzyl-2-methyl-2,3,3a,4,5,6,7,7a-octahydrobenzo[d]isoxazol-4-one which does not require peculiar preservation and storage conditions.

It is a further object to provide a salt of BTG 1640 which shows bioavailability characteristics at least comparable to those of hydrochloride salt of known BTG 1640.

The inventors of the present invention carried out studies and researches and as a result they surprisingly found out that the fumarate salt of: 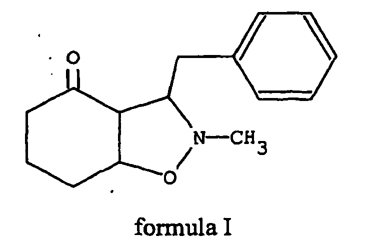 shows a highly improved stability so as not to require peculiar preservation conditions, even after six months of preservation at 40°C/75%RH.

Therefore the invention concerns fumarate salt as recited in claim 1, a new process of preparation and its use as a medicament, specifically in treating mood disorders, disorders of anxiety, depression and convulsive conditions, in the improvement of learning ability, in the reversal of amnesia, in resolving the abstinence syndrome from drugs and drugs of abuse.

Preferably, according to the invention fumarate of rel-(3R,3aS,7aS)-3-benzyl-2-methyl-2,3,3a,4,5,6,7,7a-octahydrobenzo[d]isoxazol-4-one is provided, comprising the molecule in the two enantiomeric forms and as racemic mixture.

Advantages and further characteristics are indicated in the dependent claims.

In the present invention the molecule 3-benzyl-2-methyl-2,3,3a,4,5,6,7,7a-octahydrobenzo[d]isoxazol-4-one so as represented in formula I: 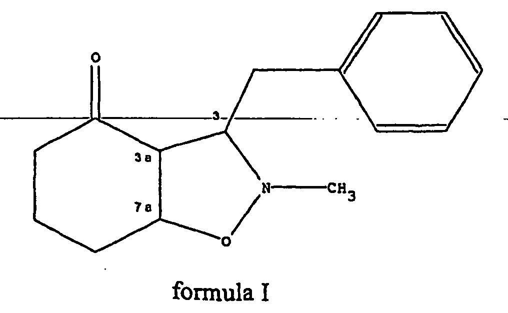 comprises in its definition any stereochemical configuration associated to chiral centers, as well as racemic mixtures and enantiomers obtainable through separation techniques known to skilled men and any mixture of two or more stereochemical compounds.

The invention will be now described in greater detail, particularly by making reference to the annexed figures wherein: ..
figure 1 is a graph of the results obtained in the stability tests carried out on BTG 1640 hydrochloride in temperature and humidity conditions of 5°C, 25°C/60% RH, 30°C/65%RH and 40°C/75%RH with reference to the purity of the examined substance;
figure 2 is a graph of the results obtained in the stability tests carried out on BTG 1640 hydrochloride in temperature and humidity conditions of 5°C, 25°C/60% RH, 30°C/65%RH and 40°C/75%RH with reference to the evaluation of total impurities;
figure 3 is a graph of the results obtained in the stability tests carried out on BTG 1640 fumarate in temperature and humidity conditions of 5°C, 25°C/60% RH, 30°C/65%RH e 40°C/75%RH with reference to the purity of the examined substance;
figure 4 is a graph of the results obtained in the stability tests carried out on BTG 1640 fumarate in temperature and humidity conditions of 5°C, 25°C/60% RH, 30°C/65%RH e 40°C/75%RH with reference to the evaluation of total impurities;
figure 5 is a graph of the results obtained in the stability tests carried out at temperatures of 5°C on BTG 1640 methanesulphonate, maleate, succinate, hydrochloride, fumarate with reference to the purity of the examined substance;
figure 6 is a graph of the results obtained in the stability tests carried out on BTG 1640 methanesulphonate, maleate, succinate, hydrochloride, fumarate at temperature of 25°C and humidity of 60%RH with reference to the purity of the examined substance;
figure 7 is a graph of the results obtained in the stability tests carried out on BTG 1640 methanesulphonate, maleate, succinate, hydrochloride, fumarate at temperature of 30°C and humidity of 65%RH with reference to the purity of the examined substance;
figure 8 is a graph of the results obtained in the stability tests carried out on BTG 1640 methanesulphonate, maleate, succinate, hydrochloride, fumarate at temperature of 40°C and humidity of 75%RH with reference to the purity of the examined substance;
figure 9 is a graph of the results obtained in the stability tests carried out at temperature of 5°C on BTG 1640 methanesulphonate, maleate, succinate, hydrochloride, fumarate with reference to the evaluation of total impurities;
figura 10 is a graph of the results obtained in the stability tests carried out at temperature of 25°C and humidity of 60%RH on BTG 1640 methanesulphonate, maleate, succinate, hydrochloride, fumarate with reference to the evaluation of total impurities;
figure 11 is a graph of the results obtained in the stability tests carried out at temperature of 30°C and humidity of 65%RH on BTG 1640 methanesulphonate, maleate, succinate, hydrochloride, fumarate with reference to the evaluation of total impurities;
figure 12 is a graph of the results obtained in the stability tests carried out at temperature of 40°C and humidity of 75%RH on BTG 1640 methanesulphonate, maleate, succinate, hydrochloride, fumarate with reference to the evaluation of total impurities.

The invention therefore concerns the fumarate salt of molecule 3-benzyl-2-methyl-2,3,3 a,4,5,6,7,7a-octahydrobenzo [d]isoxazol-4-one.

According to the invention, such salt can be obtained by treating the free base 3-benzyl-2-methyl-2,3,3a,4,5,6,7,7a-octahydrobenzo[d]isoxazol-4-one with fumaric acid, or alternatively, from hydrochloride salt of 3-benzyl-2-methyl-2,3,3a,4,5,6,7,7a-octahydrobenzo[d]isoxazol-4-one through treatment directed to free the molecule 3-benzyl-2-methyl-2,3,3a,4,5,6,7,7a-octahydrobenzo[d]isoxazol-4-one as free base and subsequent reaction with fumaric acid.

In another aspect, the invention concerns a process for the preparation of fumarate salt of 3-benzyl-2-methyl-2,3,3a,4,5,6,7,7a-octahydrobenzo[d]isoxazol-4-one according to claim 5, comprising the following steps:
i) reacting the free base 3-benzyl-2-methyl-2,3,3a,4,5,6,7,7a-octahydrobenzo[d]isoxazol-4-one with fumaric acid;
ii) subjecting the reaction mixture to one or more cooling cycles below to 10°C.

Alternatively the free base 3-benzyl-2-methyl-2,3,3a,4,5,6,7,7a-octahydrobenzo[d]isoxazol-4-one of step i) can be obtained in a preceding step to step i), which provides for freeing said base from correspondent hydrochloride salt.

Specifically, the hydrochloride salt of 3-benzyl-2-methyl-2,3,3a,4,5,6,7,7a-octahydrobenzo[d]isoxazol-4-one can be subjected to subsequent extractions in dichloromethane to obtain the molecule 3-benzyl-2-metil-2,3,3a,4,5,6,7,7a-octahydrobenzo[d]isoxazol-4-one as free base in form of a transparent oil of slightly brown colour. To such a solution, fumaric acid can be added in presence of an ice bath at about 2-8°C in form of crystals. Further preparation processes can be of course provided for by a synthesis organic technician. The salt so obtained can be furthermore optionally subjected to purification methods, if necessary.

The fumarate salt so obtained resulted to be stable in different preservation conditions, as it will be demonstrated in examples, thus demonstrating itself more stable than hydrochloride salt.

The salt fumarate of 3-benzyl-2-methyl-2,3,3a,4,5,6,7,7a-octahydrobenzo[d]isoxazol-4-one resulted, surprisingly, not only more stable than the known hydrochloride, but, as it will be widely demonstrated in the following, it resulted more bioavailable, that is it showed a kinetic absorption profile *in vivo* better than the hydrochloride salt.

Furthermore, the fumarate salt of 3-benzyl-2-methyl-2,3,3a,4,5,6,7,7a-octahydrobenzo[d]isoxazol-4-one resulted surprisingly strongly less toxic than hydrochloride salt as it will be shown below.

Fumarate salt of the compound of formula I having improved stability can be combined to suitable excipients for formulating pharmaceutical compositions according to the invention and is capable to act as pharmaceutical active substance in the treatment of mood disorders, disorders of anxiety, depression and convulsive conditions, in the improvement of learning ability, in the reversal of amnesia generated for example by Alzheimer disease or vascular dementia, in resolving the abstinence syndrome from drugs and drugs of abuse.

The daily dose required to reach the effect in the treatment of the indicated pathology varies with the subject, by depending by age, body weight and health general state, but it can be provided for a dosage suitable for the oral or topic administration in the range from 1 to 100 mg, once or more times a day, and a dosage suitable for parenteral administration in the range from 0.1 to 100 mg, once or more times a day.

The fumarate salt of compound of formula I will be added to a pharmaceutically acceptable carrier and, optionally, to other excipients in order to obtain pharmaceutical compositions to be parenterally, orally or topically administered. By the term "pharmaceutically acceptable carrier" it is meant to include solvents, supporting agents, diluents and the like, which are used as additives in order to provide a carrier suitable to the administration of the salt of the invention.

Compositions of the present invention suitable for oral administration will be conveniently in the form of discrete units such as tablets, capsules, cachets, powders, or granules, or still as suspensions in a liquid.

More preferably, the composition of the invention for the oral administration will be in form of tablets.

The tablet according to the invention comprises preferably an amount from 1 to 100 mg, preferably from 1 to 50 mg, of fumarate salt of a compound of formula I per tablet unit. Advantageously, the tablet comprises also suitable excipients, such as pre-gel starch, microcrystalline cellulose, sodium starch glycolate, talc, lactose, magnesium stearate, sucrose, stearic acid and mannitol.

Preferably, the tablet comprises from 1.9% to 41.4% by weight of fumarate of 3-benzyl-2-methyl-2,3,3a,4,5,6,7,7a-octahydrobenzo[d]isoxazol-4-one, more preferably from 2.2% to 36% by weight with respect of the total weight of the tablet.

The composition for oral administration preferably will comprise from 1 to 100 mg of fumarate salt of 3-benzyl-2-methyl-2,3,3a,4,5,6,7,7a-octahydrobenzo[d] isoxazol-4-one.

Compositions for the parenteral administration will comprise conveniently sterile aqueous preparations.

The compositions for parenteral administration preferably will comprise from 0.1 to 100 mg of fumarate salt of 3-benzyl-2-methyl-2,3,3a,4,5,6,7,7a-octahydrobenzo[d]isoxazol-4-one.

Compositions for topical administration will be conveniently in form of creams, oils, ointments, emulsions, gels, aqueous solutions, spray solutions and plasters.

The compositions for topical administration preferably will comprise from 1 to 100 mg of fumarate of 3-benzyl-2-methyl-2,3,3a,4,5,6,7,7a-octahydrobenzo[d]isoxazol-4-one.

The invention will be now described in greater details in the following examples, by way of non-limiting example of the invention and relating to the preparation of fumarate of BTG 1640 and to the evaluation of the stability, absorption efficacy and toxicity of the hydrochloride salt of the prior art and of the fumarate salt of the invention.

### Example 1. Preparation of fumarate of BTG 1640

### A. Preparation of the free base

10g of BTG 1640 hydrochloride were solubilized in 70 ml of sodium bicarbonate at 5%. The obtained solution was transferred into a separatory funnel of 250 ml and 50 ml of dichloromethane were added. After strong stirring for 30 seconds, the solution was left to rest until the separation of two layers. The phase containing dichloromethane was then transferred in an Erlenmeyer flask and further 20 ml of dichloromethane were added to the separatory funnel. Strong stirring of the solution contained in the separatory funnel was then again carried out for 30 seconds and the solution was left to rest until a new separation of the two layers. Once dichloromethane phase was separated, which was added to the Erlenmeyer flask, the extraction with further 20 ml of dichloromethane was repeated. Three aliquots of dichloromethane were then anhydrified with sodium solphate, filtered on paper filter and evaporated at rotavapor at a temperature below 35°C. The residue obtained as a slightly brown coloured oil corresponded to the free base of BTG 1640.

### B. Preparation of fumarate salt

1 g of free base BTG 1640 obtained as indicated in part A was then solubilized in 20 ml of methanol. 10 ml of a methanolic solution comprising 473 mg of fumaric acid (PM= 116.07) were then added dropwise. During the addition, the solution of BTG 1640 was kept submerged in an ice bath.

5 ml of hexane (alternatively 5 ml of ether were employed) were then added and the solution was subjected to concentration until a residue. The residue was then retaken with 36 ml of a mixture consisting of methanol/diisopropilic ether in a ratio of 1:2 and the so obtained solution was transferred into refrigerator at 2-8°C for 48 hours. At the end of 48 hours, the formed crystals were isolated through filtration and finally dried into a stove under vacuum at 35°C. Mother liquor was again concentrated to a residue and the residue was then retaken with 36 ml of diisopropilic ether. The obtained solution was then again placed into the refrigerator at 2-8°C for 48 hours. At the end of 48 hours, the formed crystals were isolated through filtration and finally dried into a stove under vacuum at 35°C. The overall yield of the process was 89%.

The so obtained crystals were subjected to characterization in order to demonstrate that they corresponded to fumarate of BTG 1640.

A sample of crystals was then subjected to elementary analysis and the obtained results corresponded to the values calculated on the basis of the proposed chemical formula.
Fumarate of BTG 1640: C₁₈H₂₂NO₄

| **Element** | **Theoric** | **Found** |
|---|---|---|
| C% | 67.31 | 67.35 |
| H% | 6.98 | 7.13 |
| N% | 4.62 | 4.43 |
| O% | 21.09 | 21.09 |

The spectrum 1H-NMR, which confirmed the formation of fumarate of BTG 1640, was then obtained.

1H-NMR (200 MHz, DMSO-d6) δ: 1.65-2.40 (m, 6H) for H5 H6 e H7; 2.41 (s, 3H) for N-CH3; 2.70-2.92 (m, 2H) for benzylic CH2; 2.92-2.98 (m, 1H) for H3a; 3.35 (br, 1H) H3; 4.25 (br, 1H) for H7a; 6.63 (s, 1H) for-CH=CH- fumarate; 7.22-7.40 (m, 5H) for aromatic structure.

### Example 2

### Analysis of the stability of hydrochloride of BTG 1640

Samples of 100 mg each of BTG 1640 hydrochloride were subjected to the following preservation conditions:
a) 5°C;
b) 25°C and 60%RH;
c) 30°C and 65%RH;
d) 40°C and 70%RH.
The purities were then analyzed periodically, initially every three months, in order to underline possible variations.

The results in the following Table 1 were obtained:

**Table 1: Purity % of BTG 1640 hydrochloride**

| Time | T=5°C | T=25°C Humidity=60%RH | T=30°C Humidity=65%RH | T=40°C Humidity=75%RH |
|---|---|---|---|---|
| 0 | 99.3 | 99.7 | 99.7 | 99.7 |
| 3 | 100.1 | 100.9 | 100.0 | 93.4 |
| 6 | 100.3 | 101.5 | 100.9 | 81.2 |
| 9 | 99.6 | 99.3 | 100.6 | |
| 12 | 99.6 | 101.1 | 99.9 | |
| 18 | 100.8 | 100.5 | | |
| 24 | 98.8 | 99.3 | | |
| 36 | 99.8 | 99.5 | | |

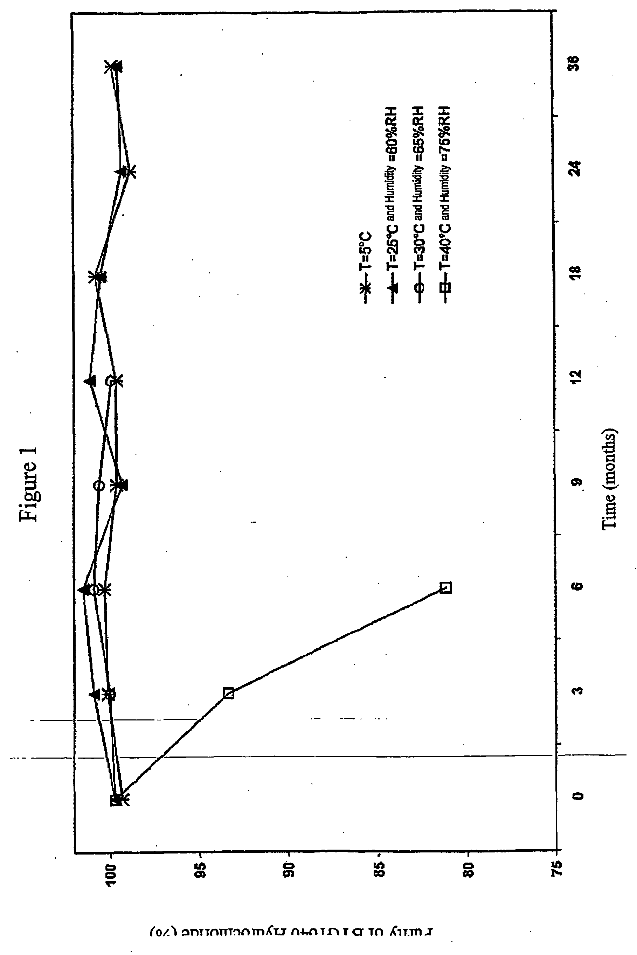

The obtained results have been represented in figure 1. As it is seen in figure 1, BTG 1640 hydrochloride showed a good stability for the T/RH conditions indicated at points a)-c) for a time period of 12 months, while it showed a strong instability since the first months, when subjected to 40°C and 75%RH, thus demonstrating that conditions d) determined a decomposition of the salt, already at six months. At the end of the test, on the basis of the results, the drug was indicated as having a validity time period of 12 months and, precautionally, preferably preserved at temperatures between 2 and 8°C, because of the strong degradation occurred in conditions 40°C/75%RH.

Contemporaneously, the impurity analysis in the conditions a)-d) of T/%RH were carried out.

The results in the following Table 2 were obtained:
**Table 2:** Calculation of impurities of BTG 1640 hydrochloride:

| Time | T=5°C | T=25°C e Humidity=60%RH | T=30°C e Humidity=65%RH | T=40°C e Humidity=75%RH |
|---|---|---|---|---|
| 0 | 0.36 | 0.36 | 0.36 | 0.36 |
| 3 | 0.58 | 0.43 | 0.44 | 6.99 |
| 6 | 0.45 | 0.87 | 0.52 | 17.31 |
| 9 | 0.31 | 0.24 | 0.12 | |
| 12 | 0.31 | 0.34 | 0.76 | |
| 18 | 0.1 | 0.13 | | |
| 24 | 0.21 | 0.28 | | |
| 36 | 0.18 | 0.30 | | |

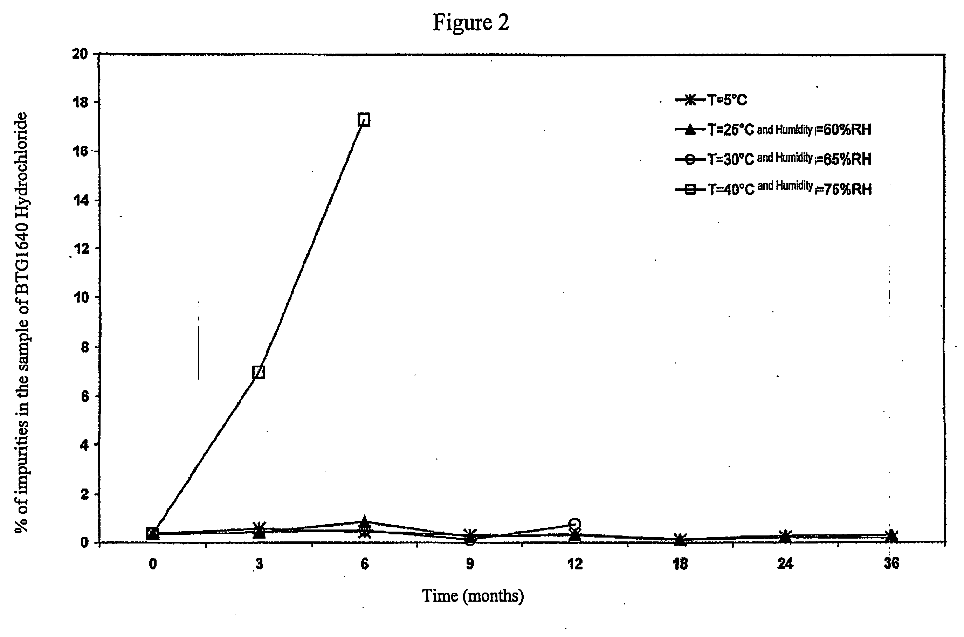

The results were then represented in figure 2, from which it was shown that BTG 1640 hydrochloride in condition d) showed, already after three months of stability at 40°C/75%RH, an unacceptable percentage of impurities.

### Example 3.

### Analysis of the stability of fumarate of BTG 1640

Samples of 100 mg each of BTG 1640 fumarate prepared according to example 1C were subjected to the following preservation conditions:
a) 5°C;
b) 25°C and 60%RH;
c) 30°C and 65%RH;
d) 40°C and 75%RH.

Quarterly and in an overall period of nine months its purity was then analyzed in order to underline possible variations. The results in the following Table 3 were obtained:
**Table 3:** Purity % of BTG 1640 fumarate

| Time | T=5°C | T=25°C Humidity=60%RH | T=30°C Humidity=65%RH | T=40°C Humidity=75%RH |
|---|---|---|---|---|
| 0 | 99.5 | 99.5 | 99.5 | 99.5 |
| 3 | 100.0.0 | 100.0 | 100.0 | 100.0 |
| 6 | 100.0 | 100.0 | 100.0 | 100.0 |
| 9 | 99.9 | 99.9 | 99.9 | |

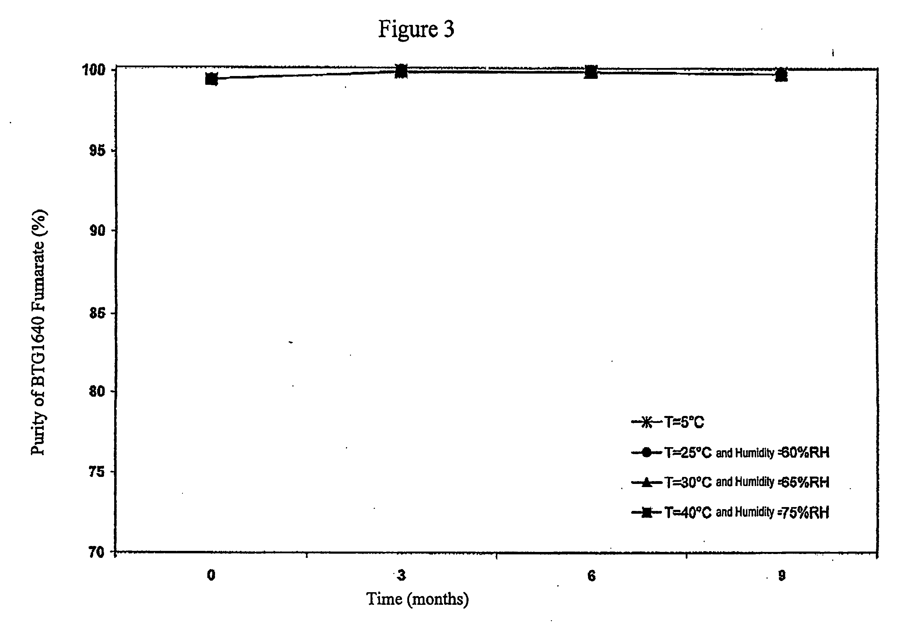

The results were then represented in figure 3. As it is seen from figure 3, BTG 1640 fumarate, similarly to BTG 1640 hydrochloride, showed a good stability for T/RH conditions indicated at points a)-c) for the overall period, and such a stability, contrary to salt BTG 1640 hydrochloride, was maintained unaltered for the overall period of observation, also in the severer condition of point d), thus demonstrating the higher stability of fumarate salt in compare to hydrochloride salt.

As a confirmation of such evidence, a test for the evaluation of the impurity percentage of the sample subjected to the same conditions a)-d) of T/%RH was parallely carried out. The experimental values in Table 4 were obtained.

**Table 4: Calculation of impurities of BTG 1640 fumarate**

| time | T=5°C | T=25°C Humidity=60%RH | T=30°C Humidity=65%RH | T=40°C Humidity=75%RH |
|---|---|---|---|---|
| 0 | 0.49 | 0.49 | 0.49 | 0.49 |
| 3 | 0.10 | 0.10 | 0.10 | 0.10 |
| 6 | 0.10 | 0.10 | 0.10 | 0.10 |
| 9 | 0.10 | 0.10 | 0.10 | |

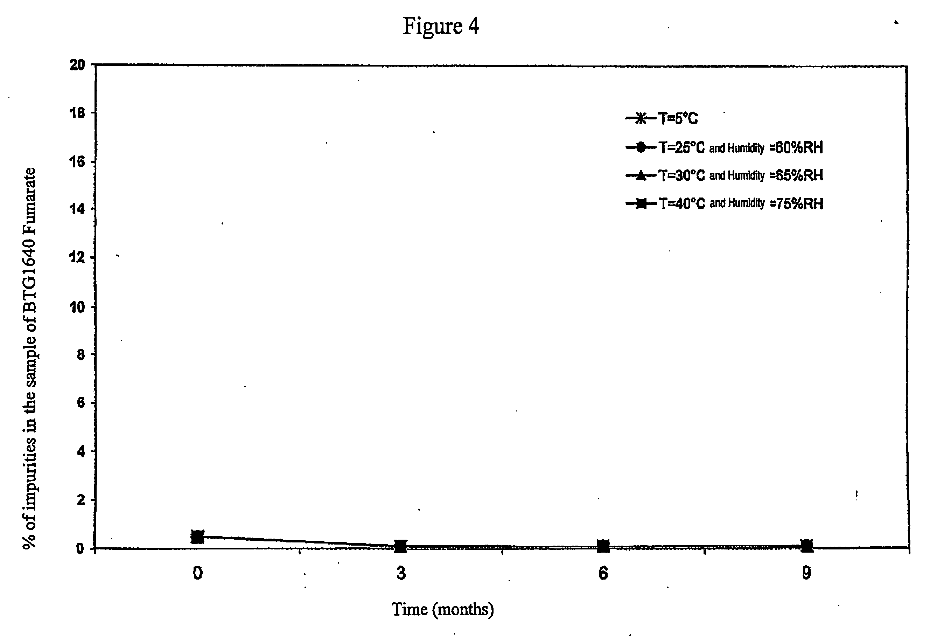

The obtained results were represented in figure 4, therefrom it is evident that BTG 1640 fumarate, also in conditions d) and until the sixth month of observation, kept to show an invariable impurity profile with respect to time zero, thus being optimal and therefore, contrary to hydrochloride, quite acceptable.

Therefore it is evident from the tests carried out the higher stability of the fumarate salt in compare to hydrochloride salt of BTG 1640.

### Example 4.

### Analysis of the stability of various salts of BTG 1640

As comparison other salts of BTG 1640 were prepared, and exactly:
- BTG 1640 methanesulphonate
- BTG 1640 maleate
- BTG 1640 succinate
following the procedure indicated in example 1 and substituting methanesolphonic, maleic and succinic acids for fumaric acid.

The stabilities were then evaluated by subjecting samples of 100 mg of each salt to the following analogous preservation conditions:
a) 5°C;
b) 25°C and 60%RH;
c) 30°C and 65%RH;
d) 40°C and 70%RH.

Quarterly and in an overall period of nine months their purity was then analyzed in order to underline possible variations.

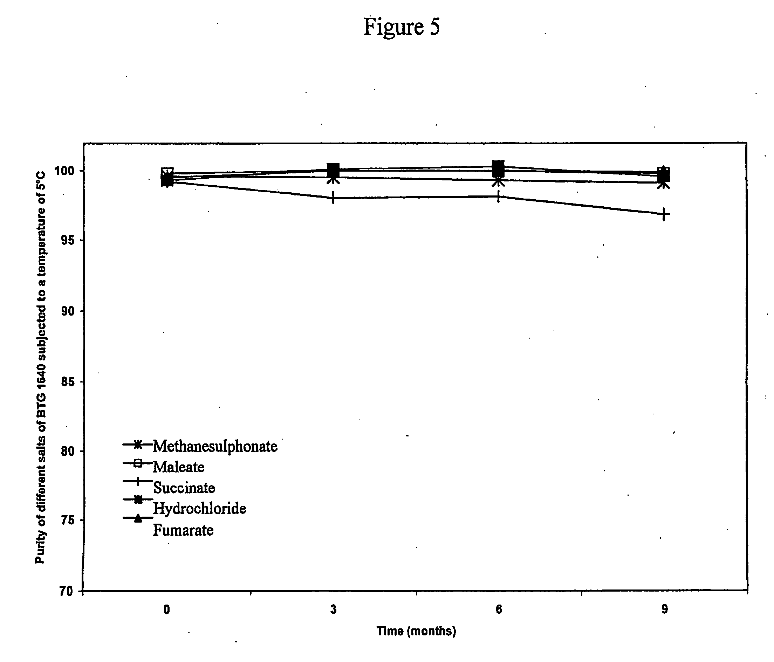

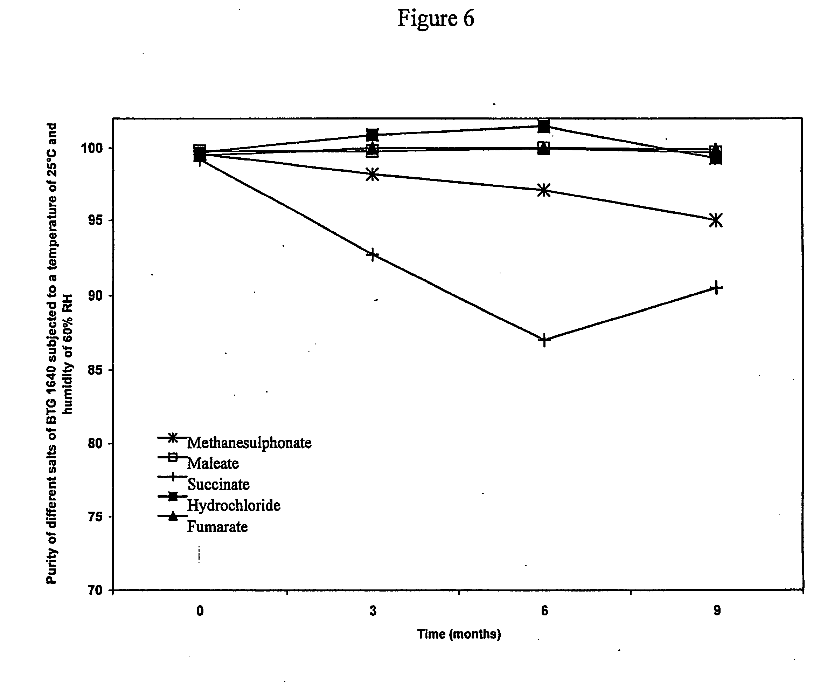

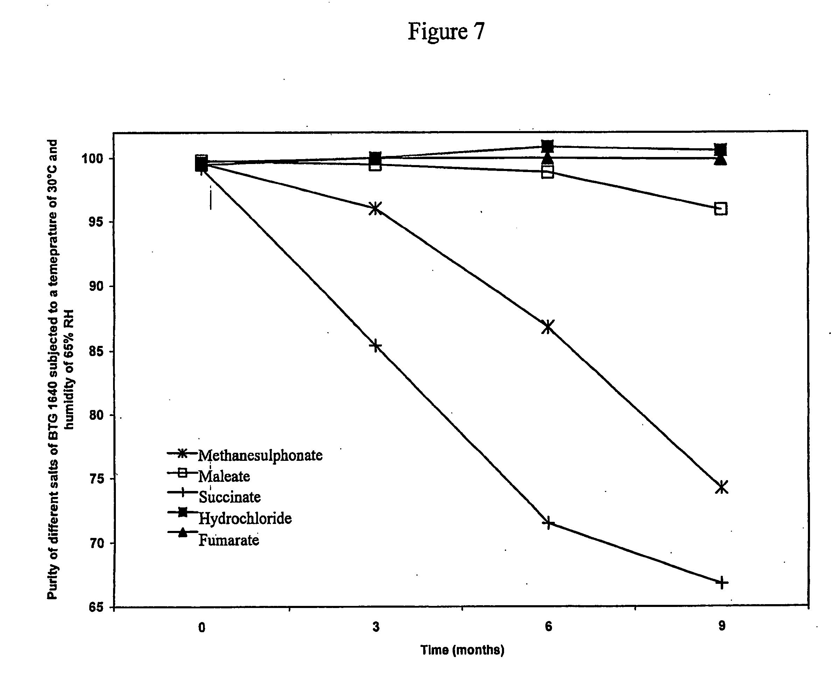

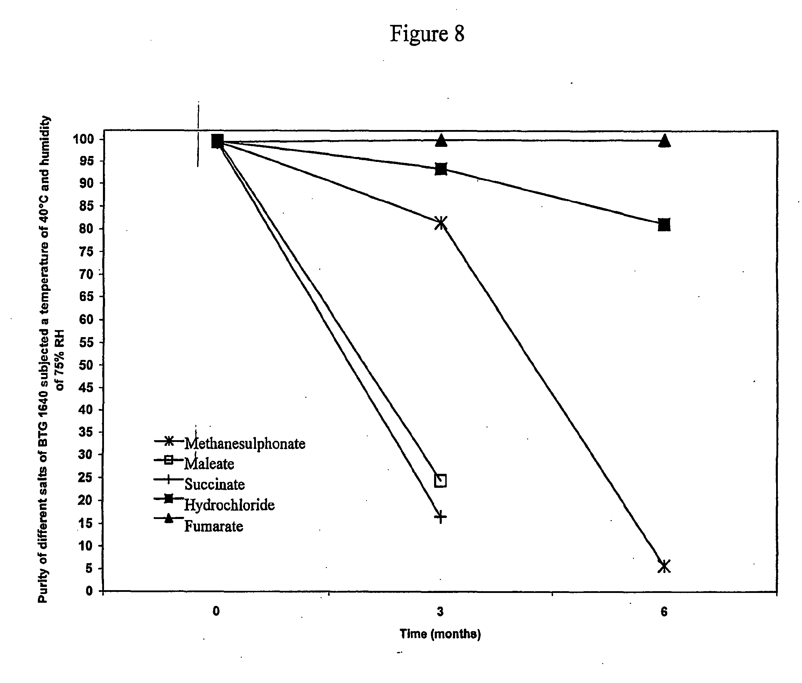

The obtained results, together with those obtained in Example 2 for hydrochloride, in Example 3 for fumarate in order to make easier the comparison, were in Table 5 and represented in figures 5-8.

**Table 5: Comparison of stabilities of different salts of BTG 1640**

| Purity data % of various salts of BTG 1640 at temperature of 5°C | | | | | |
|---|---|---|---|---|---|
| Time | Methanesulphonate | Maleate | Succinate | Hydrochloride | Fumarate |
| 0 | 99.6 | 99.8 | 99.2 | 99.3 | 99.5 |
| 3 | 99.5 | 100.0 | 98.0 | 100.1 | 100.0 |
| 6 | 99.3 | 100.0 | 98.1 | 100.3 | 100.0 |
| 9 | 99.1 | 99.8 | 96.8 | 99.6 | 99.9 |
| Purity data % of various salts of BTG 1640 at temperature of 25°C and relative humidity of 60% | | | | | |

| Time | Methanesulphonate | Maleate | Succinate | Hydrochloride | Fumarate |
|---|---|---|---|---|---|
| 0 | 99.6 | 99.8 | 99.2 | 99.7 | 99.5 |
| 3 | 98.2 | 99.8 | 92.7 | 100.9 | 100.0 |
| 6 | 97.1 | 100.0 | 87.0 | 101.5 | 100.0 |
| 9 | 95.0 | 99.7 | 90.5 | 99.3 | 99.9 |
| Purity data% of various salts of BTG 1640 at temperature of 30°C and relative humidity of 65% | | | | | |

| Time | Methanesulphonate | Maleate | Succinate | Hydrochloride | Fumarate |
|---|---|---|---|---|---|
| 0 | 99.6 | 99.8 | 99.2 | 99.7 | 99.5 |
| 3 | 96.0 | 99.5 | 85.4 | 100.0 | 100.0 |
| 6 | 86.8 | 98.9 | 71.5 | 100.9 | 100.0 |
| 9 | 74.2 | 95.9 | 66.8 | 100.6 | 99.9 |
| Purity data % of various salts of BTG 1640 at temperature of 40°C and relative humidity of 75% | | | | | |

| Time | Methanesulphonate | Maleate | Succinate | Hydrochloride | Fumarate |
|---|---|---|---|---|---|
| 0 | 99.6 | 99.8 | 99.2 | 99.7 | 99.5 |
| 3 | 81.5 | 24.4 | 16.5 | 93.4 | 100.0 |
| 6 | 5.7 | | | 81.2 | 100.0 |

As it is seen from figures 5-8 the stability profiles of salts methanesulphonate, maleate and succinate of BTG 1640 seem to be even worse than the stability profiles of BTG 1640 hydrochloride.

The methanesulphonate salt begins to degrade already after three months of stability in the cited conditions b)-d), in amount of course higher as the conditions become severer, i.e. from a) to d). Maleate salt shows an acceptable profile in conditions a) and b), while it shows considerable degradation if subjected to conditions c) already at sixth month and degrades in unacceptable way in condition d). Succinate salt, at the end, shows degradation at all observation conditions a)-d), already from the third month and considerably as higher as severer are preservation conditions, i.e. from a) to d).

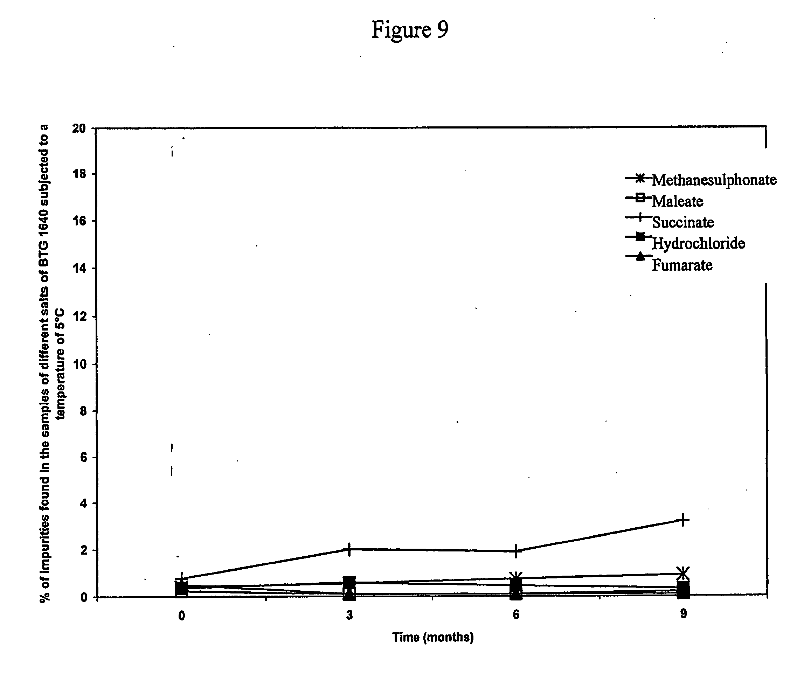

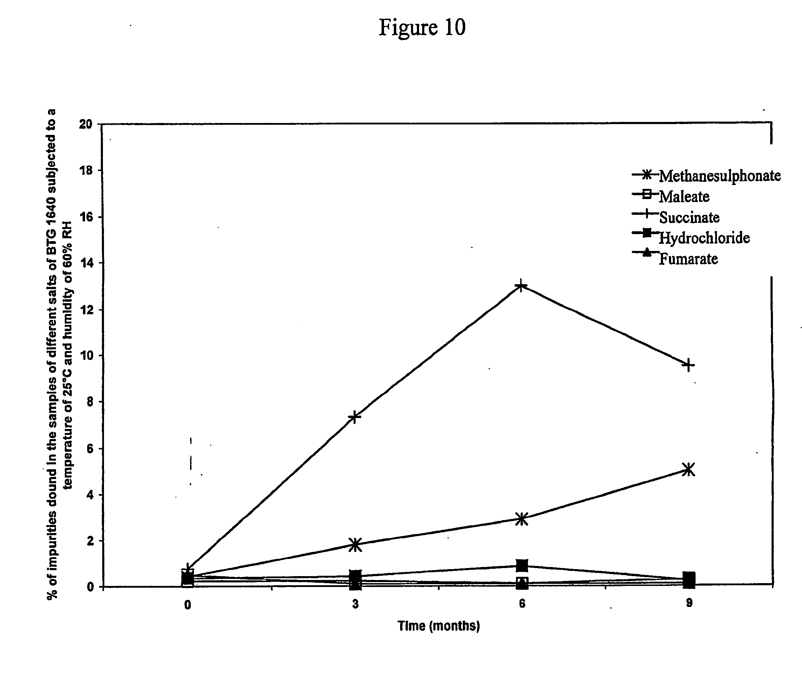

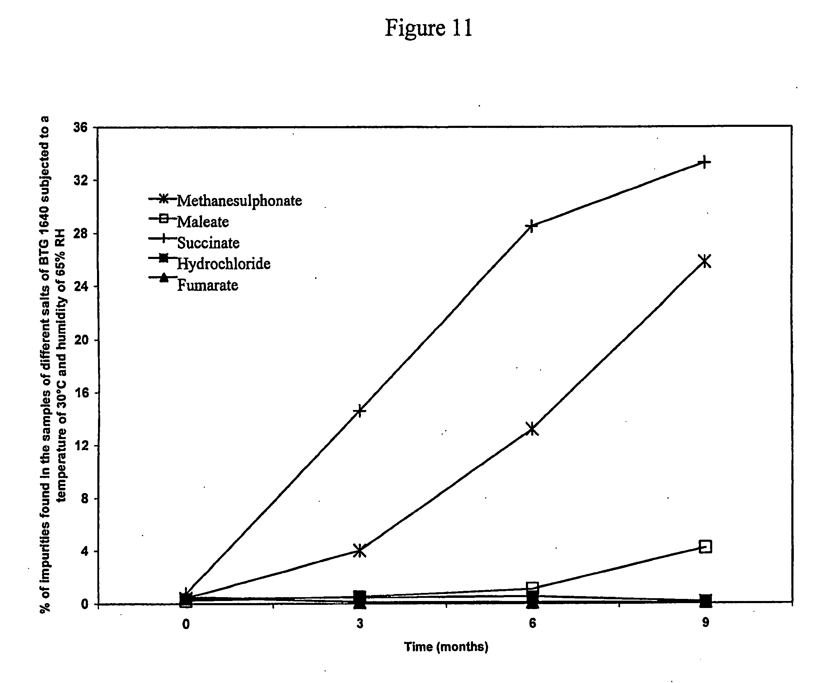

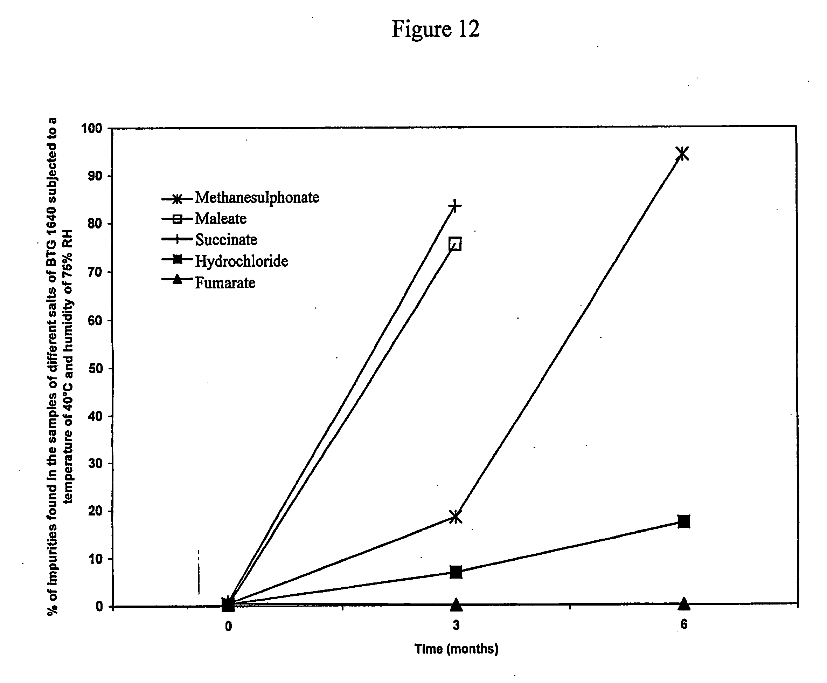

Consistently to these results, the impurity profile of salt samples subjected to such preservation conditions showed an unacceptable increase of the same as the salt itself degraded, as shown by patterns represented in figures 9-12, obtained on the basis of impurity results in Table 6.

**Table 6: Comparison of impurity amount of various salts of BTG 1640**

| **% impurities of various salts of BTG 1640 subjected to temperature of 5°C** | | | | | |
|---|---|---|---|---|---|
| Time | Methanesulphonate | Maleate | Succinate | Hydrochloride | Fumarate |
| 0 | 0.42 | 0.23 | 0.77 | 0.36 | 0.49 |
| 3 | 0.55 | 0.10 | 2.00 | 0.58 | 0.10 |
| 6 | 0.74 | 0.10 | 1.90 | 0.45 | 0.10 |
| 9 | 0.91 | 0.19 | 3.20 | 0.31 | 0.10 |
| **% impurities of various salts of BTG 1640 subjected t temperature of 25°C and relative humidity of 60%** | | | | | |

| Time | Methanesulphonate | Maleate | Succinate | Hydrochloride | Fumarate |
|---|---|---|---|---|---|
| 0 | 0.42 | 0.23 | 0.77 | 0.36 | 0.49 |
| 3 | 1.80 | 0.22 | 7.30 | 0.43 | 0.10 |
| 6 | 2.90 | 0.10 | 13.00 | 0.87 | 0.10 |
| 9 | 5.00 | 0.29 | 9.50 | 0.24 | 0.10 |
| **% impurities of various salts of BTG 1640 subjected to temperature of 30°C and relative humidity of 65%** | | | | | |

| Time | Methanesulphonate | Maleate | Succinate | Hydrochloride | Fumarate |
|---|---|---|---|---|---|
| 0 | 0.42 | 0.23 | 0.77 | 0.36 | 0.49 |
| 3 | 4.00 | 0.49 | 14.60 | 0.44 | 0.10 |
| 6 | 13.20 | 1.10 | 28.50 | 0.52 | 0.10 |
| 9 | 25.80 | 4.20 | 33.30 | 0.12 | 0.10 |
| **% impurities of various salts of BTG 1640 subjected to temperature of 40°C and relative humidity of 75%** | | | | | |
| 0 | 0.42 | 0.23 | 0.77 | 0.36 | 0.49 |
| 3 | 18.50 | 75.60 | 83.50 | 6.99 | 0.10 |
| 6 | 94.30 | | | 17.31 | 0.10 |

Surprisingly, therefore fumarate showed a different and improved stability with respect to known hydrochloride and other prepared acid addition salts, thus turning out to be the best ingredient for formulating pharmaceutical compositions, which does not require peculiar preservation conditions.

The present invention succeeded in solving the technical problem of obtaining a form of stable BTG 1640 through identification of the fumarate salt.

### Example 5.

### Analysis of absorption kinetic of fumarate and hydrochloride of BTG 1640

Absorption extent and rate of molecule BTG 1640 in its forms of fumarate and hydrochloride salts were evaluated.

With this intent, the two salts were dispersed in an aqueous suspension of arabic gum at 5% thus obtaining two formulations, each one orally administered through oesophageous gavage at dose of 10 mg/kg (expressed as free base of BTG 1640) to five rats Sprague Dawley.

Plasma concentration of molecule BTG 1640 was determined at different time points.

The results in Table 7 were obtained.

**Table 7: Concentration of fumarate and hydrochloride in Plasma**

| *Time (minutes)* | BTG 1640 free base (µg/mL) with Fumarate Salt Administration | BTG 1640 free base (µg/mL) with Hydrochloride Salt Administration |
|---|---|---|
| 2 | 1.06728 | 0.7402 |
| 5 | 2.35406 | 1.4242 |
| 30 | 0.83756 | 0.2354 |
| 90 | 0 | 0.1184 |
| 300 | 0 | 0 |

From the table 7, it is evident that fumarate, even if provides a not significantly higher concentration of free and unchanged active principle BTG 1640 with respect to hydrochloride after two minutes from its administration, it provides a much higher concentration of free base after 5 minutes with respect to hydrochloride.

All in all, the availability of the active principle in the circulation is more favourable after administration of fumarate salt thus it can be expected an higher pharmacological activity.

### Example 6

### Evaluation of acute oral toxicity

Acute oral toxicity of salts hydrochloride and fumarate of BTG-1640 was evaluated.

The two salts were dispersed in an aqueous suspension, thus obtaining two formulations, each one orally administered through oesophageous gavage at dose of 2000 mg/kg bw (expressed as free base of BTG 1640) to one of two groups comprising three female CD-1 mice per group.

The observation lasted 14 days.

In all the animals of each group a reduction of the spontaneous locomotory activity was recorded in the first minutes after the treatment and quickly disappeared. Lethargy, ataxia, aggressiveness and vocalization were observed in the mice treated with hydrochloride BTG 1640 and two of them died in the first day following the administration of the entire dose. The body weight remained quite stable over the entire period of observation in all the experimental groups. Necropsy revealed the absence of alterations, anyway kidneys were sampled from each animal, in order to assess the possible renal toxicity of the salts. The histopathological examination of the group administered with hydrochloride showed small foci of acute tubular necrosis in the cortex, balloon degeneration of the epithelium and dilatation of convoluted tubules. In the group administered with fumarate only a mild dilatation of convoluted tubules was observed. On the basis of the discussed results, the administration of 2000 mg/kg bw, in female mice, of fumarate BTG 1640 resulted strongly less toxic, being followed by quickly reversible neurological signs, while the same dose of hydrochloride BTG 1640 was lethal in two animals out of three.

Some formulative examples for the preparation of tablets having weight of about 120 mg of pharmaceutical composition according to the invention comprising fumarate salt of BTG 1640 and directed to oral administration are indicated in the following.

### Example 7

| | |
|---|---|
| BTG 1640 fumarate | 2.7 mg |
| Sucrose | 81.3 mg |
| Microcrystalline cellulose | 20.4 mg |
| Talc | 9.6 mg |
| Stearic acid | 6.0 mg |

### Example 8

| | |
|---|---|
| BTG 1640 fumarate | 5.4 mg |
| Sucrose | 78.6 mg |
| Microcrystalline cellulose | 20.4 mg |
| Talc | 9.6 mg |
| Stearic acid | 6.0 mg |

### Example 9

| | |
|---|---|
| BTG 1640 fumarate | 10.8 mg |
| Sucrose | 73.2 mg |
| Microcrystalline cellulose | 20.4 mg |
| Talc | 9.6 mg |
| Stearic acid | 6.0 mg |

### Example 10

| | |
|---|---|
| BTG 1640 fumarate | 21.6 mg |
| Sucrose | 62.4 mg |
| Microcrystalline cellulose | 20.4 mg |
| Talc | 9.6 mg |
| Stearic acid | 6.0 mg |

### Example 11

| | |
|---|---|
| BTG 1640 fumarate | 43.2 mg |
| Sucrose | 40.8 mg |
| Microcrystalline cellulose | 20.4 mg |
| Talc | 9.6 mg |
| Stearic acid | 6.0 mg |

### Example 12

| | |
|---|---|
| BTG 1640 fumarate | 2.7 mg |
| Pre-gel starch | 24.0 mg |
| Microcrystalline cellulose | 76.5 mg |
| Sodium starch-glycolate | 4.8 mg |
| Talc | 12.0 mg |

### Example 13

| | |
|---|---|
| BTG 1640 fumarate | 5.4 mg |
| Pre-gel starch | 24.0 mg |
| Microcrystalline cellulose | 73.8 mg |
| Sodium starch glycolate | 4.8 mg |
| Talc | 12.0 mg |

### Example 14

| | |
|---|---|
| BTG 1640 fumarate | 10.8 mg |
| Pre-gel starch | 24.0 mg |
| Microcrystalline cellulose | 68.4 mg |
| Sodium starch glycolate | 4.8 mg |
| Talc | 12.0 mg |

### Example 15

| | |
|---|---|
| BTG 1640 fumarate | 21.6mg |
| Pre-gel starch | 24.0 mg |
| Microcrystalline cellulose | 57.6 mg |
| Sodium starch glycolate | 4.8 mg |
| Talc | 12.0 mg |

### Example 16

| | |
|---|---|
| BTG 1640 fumarate | 43.2 mg |
| Pre-gel-starch | 24.0mg |
| Microcrystalline cellulose | 36.0 mg |
| Sodium starch glycolate | 4.8 mg |
| Talc | 12.0 mg |

### Example 17

| | |
|---|---|
| BTG 1640 fumarate | 2.7 mg |
| Lactose | 73.8 mg |
| Microcrystalline cellulose | 42.9 mg |
| Magnesium Stearate | 0.6 mg |

### Example 18

| | |
|---|---|
| BTG 1640 fumarate | 5.4 mg |
| Lactose | 73.8 mg |
| Microcrystalline cellulose | 40.2 mg |
| Magnesium Stearate | 0.6 mg |

### Example 19

| | |
|---|---|
| BTG 1640 fumarate | 10.8 mg |
| Lactose | 73.8 mg |
| Microcrystalline cellulose | 34.8 mg |
| Magnesium Stearate | 0.6 mg |

### Example 20

| | |
|---|---|
| BTG 1640 fumarate | 21.6 mg |
| Lactose | 73.8 mg |
| Microcrystalline cellulose | 24.0 mg |
| Magnesium Stearate | 0.6 mg |

### Example 21

| | |
|---|---|
| BTG 1640 fumarate | 43.2 mg |
| Lactose | 73.8 mg |
| Microcrystalline cellulose | 2.4 mg |
| Magnesium Stearate | 0.6 mg |

### Example 22

| | |
|---|---|
| BTG 1640 fumarate | 2.7 mg |
| Mannitol | 24.0 mg |
| Microcrystalline cellulose | 76.5 mg |
| Sodium starch glycolate | 4.8 mg |
| Talc | 12.0 mg |

### Example 23

| | |
|---|---|
| BTG 1640 fumarate | 5.4 mg |
| Mannitol | 24.0 mg |
| Microcrystalline cellulose | 73.8 mg |
| Sodium starch glycolate | 4.8 mg |
| Talc | 12.0 mg |

### Example 24

| | |
|---|---|
| BTG 1640 fumarate | 10.8 mg |
| Mannitol | 24.0 mg |
| Microcrystalline cellulose | 68.4 mg |
| Sodium starch glycolate | 4.8 mg |
| Talc | 12.0 mg |

### Example 25

| | |
|---|---|
| BTG 1640 fumarate | 21.6 mg |
| Mannitol | 24.0 mg |
| Microcrystalline cellulose | 57.6 mg |
| Sodium starch glycolate | 4.8 mg |
| Talc | 12.0 mg |

### Example 26

| | |
|---|---|
| BTG 1640 fumarate | 43.2 nig |
| Mannitol | 24.0 mg |
| Microcrystalline cellulose | 36.0 mg |
| Sodium starch glycolate | 4.8 mg |
| Talc | 12.0 mg |

## Claims

1. Fumarate salt of the compound of formula I

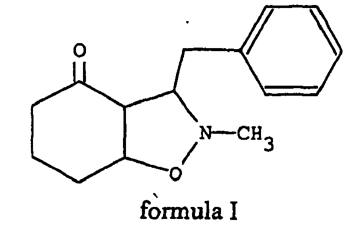

2. The fumarate salt of claim 1 which is fumarate salt of the compound of formula I in the form of one or more stereochemical compounds and mixtures thereof.

3. The fumarate salt of claim 2 which is fumarate salt of the compound of formula I in the form of a diastereoisomer or a racemic mixture.

4. The fumarate salt according to any one of claims 1-3, which is fumarate of rel-(3R,3aS,7aS)-3-benzyl-2-methyl-2,3,3a,4,5,6,7,7a-octahydrobenzo[d]isoxazol-4-one.

5. A process for preparing fumarate of 3-benzyl-2-methyl-2,3,3a,4,5,6,7,7a-octahydrobenzo[d]isoxazol-4-one, according to any one of claims 1-4, comprising the following steps:
i) reacting the free base 3-benzyl-2-methyl-2,3,3a,4,5,6,7,7a-octahydrobenzo[d]isoxazol-4-one with fumaric acid;
ii) subjecting the reaction mixture to one or more cooling cycles of less than 10°C.

6. The process according to claim 5, wherein the free base 3-benzyl-2-methyl-2,3,3a,4,5,6,7,7a-octahydrobenzo[d]isoxazol-4-one of step i) is obtained in a step being previous to step i), which provides for freeing the said base from the corresponding hydrochloride salt.

7. The process according to claim 6, wherein freeing of the free base from the hydrochloride salt is carried out by dichloromethane.

8. The process according to any one of claims 5-7, wherein the step i) is carried out in an ice bath.

9. The process according to any one of claims 5-8, wherein the cooling of step ii) occurs at temperatures from 2 to 8°C.

10. Fumarate salt of the compound of formula I 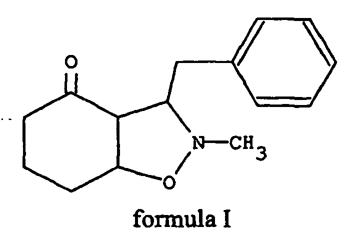 for use as a medicament.

11. The fumarate salt of claim 10 which is fumarate salt of the compound of formula I in the form of one or more stereochemical compounds and mixtures thereof for use as a medicament.

12. The fumarate salt of claim 11 which is fumarate salt of the compound of formula I in the form of a diastereoisomer or a racemic mixture for use as a medicament.

13. The fumarate salt according to any one of claims 10-12, which is fumarate of rel-(3R,3aS,7aS)-3-benzyl-2-methyl-2,3,3a,4,5,6,7,7a-octahydrobenzo[d]isoxazol-4-one for use as a medicament.

14. A pharmaceutical composition comprising the fumarate salt according to any one of claims 1-4 and a pharmaceutically acceptable carrier.

15. The composition according to claim 14, wherein the fumarate salt is present in an amount of from 0.1 to 100 mg.

16. A tablet comprising the fumarate salt according to any one of claims 1-4 and suitable excipients.

17. The tablet according to claim 16, wherein the suitable excipients are selected from the group consisting of pre-gel starch, microcrystalline cellulose, sodium starch glycolate, talc, lactose, magnesium stearate, saccarose, stearic acid and mannitol.

18. The tablet according to claim 16 or claim 17, wherein the fumarate salt is present in an amount of from 1 to 100 mg per tablet unit.

19. The tablet according to claim 18, wherein the fumarate salt is present in an amount of from 1 to 50 mg per tablet unit.

20. Use of the fumarate salt of the compound of formula I 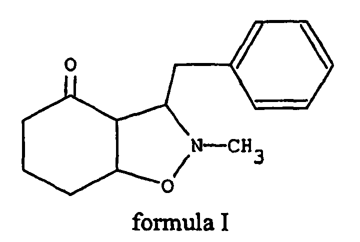 for the manufacture of a medicament for treating mood disorders, disorders of anxiety, depression and convulsive conditions, in the improvement of learning ability, in the reversal of amnesia, in resolving the abstinence syndrome from drugs and drugs of abuse.

21. The use according to claim 20, wherein the fumarate salt is fumarate of the compound of formula I in the form of one or more stereochemical compounds and mixtures thereof for the manufacture of a medicament for treating mood disorders, disorders of anxiety, depression and convulsive conditions, in the improvement of learning ability, in the reversal of amnesia, in resolving the abstinence syndrome from drugs and drugs of abuse.

22. The use according to claim 21, wherein the fumarate salt is fumarate of the compound of formula I in the form of a diastereoisomer or a racemic mixture for the manufacture of a medicament for treating mood disorders, disorders of anxiety, depression and convulsive conditions, in the improvement of learning ability, in the reversal of amnesia, in resolving the abstinence syndrome from drugs and drugs of abuse.

23. The use according to any one of claims 20-22, wherein the fumarate salt is fumarate of rel-(3R,3aS,7aS)-3-benzyl-2-methyl-2,3,3a,4,5,6,7,7a-octahydrobenzo[d]isoxazol-4-one for the manufacture of a medicament for treating mood disorders, disorders of anxiety, depression and convulsive conditions, in the improvement of learning ability, in the reversal of amnesia, in resolving the abstinence syndrome from drugs and drugs of abuse.

24. The use according to any one of claims 21-23, wherein the medicament is for oral administration.

25. The use according to claim 24, wherein the medicament for oral administration is selected from the group consisting of tablets, capsules, cachets, powders, granules, or suspensions in a liquid.

26. The use according to claim 24 or claim 25, wherein the fumarate salt of the compound of formula I is in an amount of from 1 to 100 mg per medicament unit.

27. The use according to claim 26, wherein the medicament is a tablet, where the fumarate salt of the compound of formula I is in an amount of from 1 to 100 mg per tablet unit.

28. The use according to any one of claims 20-23, wherein the medicament is for topical administration.

29. The use according to claim 28, wherein the medicament for topical administration is selected from the group consisting of creams, oils, ointments, emulsions, gels, aqueous solutions, spray solutions and plasters.

30. The use according to claim 28 or claim 29, wherein the fumarate salt of the compound of formula I is in an amount of from 1 to 100 mg per medicament unit.

31. The use according to any one of claims 20-23, wherein the medicament is for parenteral administration.

32. The use according to claim 31, wherein the medicament for parenteral administration is a sterile aqueous preparation.

33. The use according to claim 31 or claim 32, wherein the fumarate salt of the compound of formula I is in an amount of from 0.1 to 100 mg per medicament unit.

34. Fumarate salt of the compound of formula I 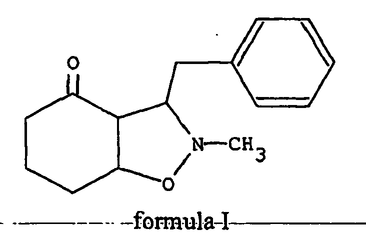 for use in the treatment of mood disorders, disorders of anxiety, depression and convulsive conditions, in the improvement of learning ability, in the reversal of amnesia, in resolving the abstinence syndrome from drugs and drugs of abuse.

## Patentansprüche

1. Fumarat-Salz der Verbindung der Formel I

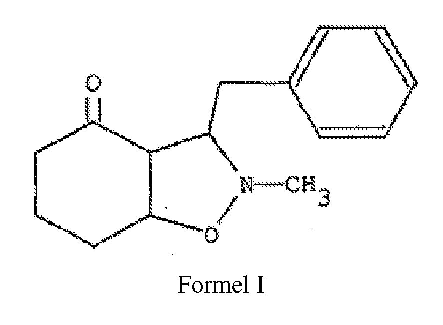

2. Fumarat-Salz nach Anspruch 1, welches das Fumarat-Salz der Verbindung der Formel I in Form einer oder mehrerer stereochemischer Verbindungen und Mischungen daraus ist.

3. Fumarat-Salz nach Anspruch 2, welches das Fumarat-Salz der Verbindung der Formel I in Form eines Diastereoisomers oder einer racemischen Mischung ist.

4. Fumarat-Salz nach irgendeinem der Ansprüche 1 bis 3, welches das Fumarat von rel-(3R,3aS,7aS)-3-Benzyl-2-methyl-2,3,3a,4,5,6,7,7a-octahydrobenzo[d]-isoxazol-4-on ist.

5. Verfahren zur Herstellung des Fumarats von 3-Benzyl-2-methyl-2,3,3a,4,5,6,7,7a-octahydrobenzo[d]isoxazol-4-on nach irgendeinem der Ansprüche 1 bis 4, umfassend die folgenden Schritte:
i) Umsetzen der freien Base 3-Benzyl-2-methyl-2,3,3a,4,5,6,7,7a-octahydrobenzo[d]isoxazol-4-on mit Fumarsäure;
ii) Aussetzen der Reaktionsmischung eines oder mehreren Kühlzyklen bei einer Temperatur von weniger als 10 °C.

6. Verfahren nach Anspruch 5, worin die freie Base 3-Benzyl-2-methyl-2,3,3a,4,5,6,7,7a-octahydrobenzo[d]isoxazol-4-on von Schritt i) erhalten wird in einem Schritt, der Schritt i) vorgelagert ist, der ein Freisetzen der Base von dem entsprechenden Hydrochloridsalz liefert.

7. Verfahren nach Anspruch 6, worin das Freisetzen der freien Base aus dem Hydrochloridsalz durchgeführt wird durch Dichlormethan.

8. Verfahren nach irgendeinem der Ansprüche 5 bis 7, worin der Schritt i) durchgeführt wird in einem Eisbad.

9. Verfahren nach irgendeinem der Ansprüche 5 bis 8, worin das Kühlen des Schritts ii) bei einer Temperatur von 2 bis 8 °C stattfindet.

10. Fumarat-Salz der Verbindung der Formel I 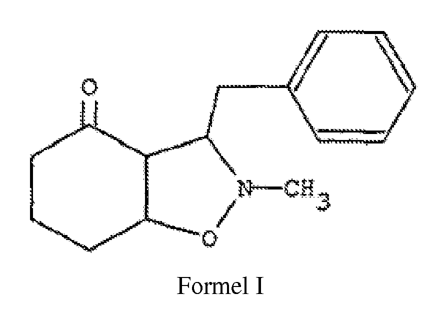 zur Verwendung als Medikament.

11. Fumarat-Salz nach Anspruch 10, welches ein Fumarat-Salz der Verbindung der Formel I in Form einer oder mehrerer stereochemischer Verbindungen und Mischungen daraus ist, zur Verwendung als Medikament.

12. Fumarat-Salz nach Anspruch 11, welches das Fumarat-Salz der Verbindung der Formel I in Form eines Diastereoisomers oder einer racemischen Mischung ist, zur Verwendung als Medikament.

13. Fumarat-Salz nach irgendeinem der Ansprüche 10 bis 12, welches das Fumarat-Salz von rel-(3R,3aS,7aS)-3-Benzyl-2-methyl-2,3,3a,4,5,6,7,7a-octahydrobenzo-[d]isoxazol-4-on ist, zur Verwendung als Medikament.

14. Pharmazeutische Zubereitung, umfassend das Fumarat-Salz nach irgendeinem der Ansprüche 1 bis 4, und einen pharmazeutisch annehmbaren Träger.

15. Zubereitung nach Anspruch 14, worin das Fumarat-Salz in einer Menge von 0,1 bis 100 mg zugegen ist.

16. Tablette, umfassend das Fumarat-Salz nach irgendeinem der Ansprüche 1 bis 4, und geeignete Träger.

17. Tablette nach Anspruch 16, worin die geeigneten Träger gewählt sind aus der Gruppe, die besteht aus Pre-Gel-Stärke, mikrokristalliner Cellulose, Natriumstärke-Glycolat, Talkum, Lactose, Magnesiumstearat, Saccarose, Stearinsäure und Mannitol.

18. Tablette nach Anspruch 16 oder Anspruch 17, worin das Fumarat-Salz zugegen ist in einer Menge von 1 bis 100 mg pro Tabletteneinheit.

19. Tablette nach Anspruch 18, worin das Fumarat-Salz zugegen ist in einer Menge von 1 bis 50 mg pro Tabletteneinheit.

20. Verwendung des Fumarat-Salzes der Verbindung der Formel I 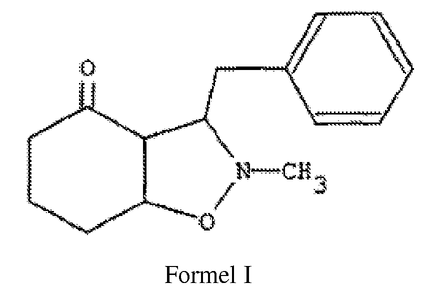 für die Herstellung eines Medikaments zur Behandlung von Stimmungsstörungen, Störungen aus Angst, Depressionen und konvulsiven Zuständen, bei der Verbesserung des Lernvermögens, bei der Umkehr von Amnesie, beim Lösen des Abstinenz-Syndroms von Arzneimitteln und Arzneimittelmissbrauch.

21. Verwendung nach Anspruch 20, worin das Fumarat-Salz das Fumarat der Verbindung der Formel I in Form einer oder mehrerer sterochemischer Verbindungen und Mischungen daraus ist, für die Herstellung eines Medikaments zur Behandlung von Stimmungsstörungen, Störungen aus Angst, Depressionen und konvulsiven Zuständen, bei der Verbesserung des Lernvermögens, bei der Umkehr von Amnesie, beim Lösen des Abstinenz-Syndroms von Arzneimitteln und Arzneimittelmissbrauch.

22. Verwendung nach Anspruch 21, worin das Fumarat-Salz das Fumarat der Verbindung der Formel I in Form eines Diastereoisomers oder einer racemischen Mischung ist, für die Herstellung eines Medikaments zur Behandlung von Stimmungsstörungen, Störungen aus Angst, Depressionen und konvulsiven Zuständen, bei der Verbesserung des Lernvermögens, bei der Umkehr von Amnesie, beim Lösen des Abstinenz-Syndroms von Arzneimitteln und Arzneimittelmissbrauch.

23. Verwendung nach irgendeinem der Ansprüche 20 bis 22, worin das Fumarat-Salz das Fumarat von rel-(3R,3aS,7aS)-3-Benzyl-2-methyl-2,3,3a,4,5,6,7,7a-octahydrobenzo[d]isoxazol-4-on ist, für die Herstellung eines Medikaments zur Behandlung von Stimmungsstörungen, Störungen aus Angst, Depressionen und konvulsiven Zuständen, bei der Verbesserung des Lernvermögens, bei der Umkehr von Amnesie, beim Lösen des Abstinenz-Syndroms von Arzneimitteln und Arzneimittelmissbrauch.

24. Verwendung nach irgendeinem der Ansprüche 21 bis 23, worin das Medikament ein Medikament für eine orale Verabreichung ist.

25. Verwendung nach Anspruch 24, worin das Medikament zur oralen Verabreichung gewählt ist aus der Gruppe, die besteht aus Tabletten, Kapseln, Briefchen, Pulvern, Granulaten oder Suspensionen in einer Flüssigkeit.

26. Verwendung nach Anspruch 24 oder Anspruch 25, worin das Fumarat-Salz der Verbindung der Formel I in einer Menge von 1 bis 100 mg pro Medikamenten-Einheit vorliegt.

27. Verwendung nach Anspruch 26, worin das Medikament eine Tablette ist, worin das Fumarat-Salz der Verbindung der Formel I in einer Menge von 1 bis 100 mg pro Tabletteneinheit vorliegt.

28. Verwendung nach irgendeinem der Ansprüche 20 bis 23, worin das Medikament ein Medikament zur topischen Verabreichung ist.

29. Verwendung nach Anspruch 28, worin das Medikament zur topischen Verabreichung gewählt ist aus der Gruppe, die besteht aus Cremes, Ölen, Salben, Emulsionen, Gelen, wässrigen Lösungen, Sprühlösungen und Pflastern.

30. Verwendung nach Anspruch 28 oder Anspruch 29, worin das Fumarat-Salz der Verbindung der Formel I in einer Menge von 1 bis 100 mg pro Medikamenten-Einheit vorliegt.

31. Verwendung nach irgendeinem der Ansprüche 20 bis 23, worin das Medikament ein Medikament zur parenteralen Verabreichung ist.

32. Verwendung nach Anspruch 31, worin das Medikament zur parenteralen Verabreichung eine sterile wässrige Zubereitung ist.

33. Verwendung nach Anspruch 31 oder Anspruch 32, worin das Fumarat-Salz der Verbindung der Formel I in einer Menge von 0,1 bis 100 mg pro Medikamenten-Einheit vorliegt.

34. Fumarat-Salz der Verbindung der Formel I 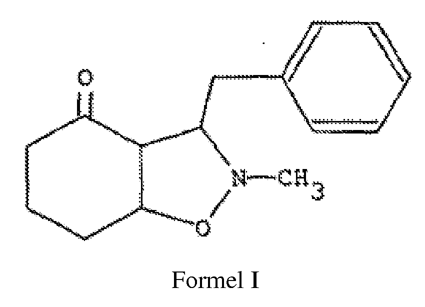 zur Verwendung bei der Behandlung von Stimmungsstörungen, Störungen aus Angst, Depressionen und konvulsiven Zuständen, bei der Verbesserung des Lernvermögens, bei der Umkehr von Amnesie, beim Lösen des Abstinenz-Syndroms von Arzneimitteln und Arzneimittelmissbrauch.

## Revendications

1. Sel de fumarate du composé de formule I :

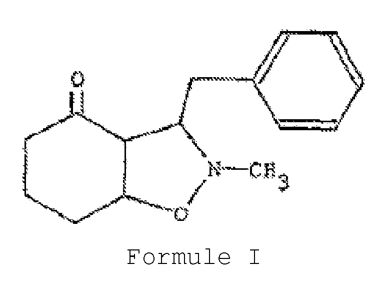

2. Sel de fumarate selon la revendication 1, qui est un sel de fumarate du composé de formule I sous la forme d'un ou de plusieurs composés stéréochimiques et de leurs mélanges.

3. Sel de fumarate selon la revendication 2, qui est un sel de fumarate du composé de formule I sous la forme d'un diastéréoisomère ou d'un mélange racémique.

4. Sel de fumarate selon l'une quelconque des revendications 1 à 3, qui est le fumarate de rel-(3R,3aS,7aS)-3-benzyl-2-méthyl-2,3,3a,4,5,6,7,7a-octahydrobenzo[d]isoxazol-4-one.

5. Procédé de préparation du fumarate de 3-benzyl-2-méthyl-2,3,3a,4,5,6,7,7a-octahydrobenzo[d]isoxazol-4-one, selon l'une quelconque des revendications 1 à 4, comprenant les étapes suivantes :
i) faire réagir la base libre 3-benzyl-2-méthyl-2,3,3a,4,5,6,7,7a-octahydrobenzo[d]isoxazol-4-one avec de l'acide fumarique ;
ii) soumettre le mélange réactionnel à un ou plusieurs cycles de refroidissement à moins de 10°C.

6. Procédé selon la revendication 5, dans lequel la base libre 3-benzyl-2-méthyl-2,3,3a,4,5,6,7,7a-octahydrobenzo[d]isoxazol-4-one de l'étape i) est obtenue dans une étape antérieure à l'étape i), qui permet la libération de ladite base à partir du chlorhydrate correspondant.

7. Procédé selon la revendication 6, dans lequel la libération de la base libre à partir du chlorhydrate est effectuée dans le dichlorométhane.

8. Procédé selon l'une quelconque des revendications 5 à 7, dans lequel l'étape i) est menée dans un bain de glace.

9. Procédé selon l'une quelconque des revendications 5 à 8, dans lequel le refroidissement de l'étape ii) a lieu à des températures allant de 2 °C à 8°C.

10. Sel de fumarate du composé de formule I : 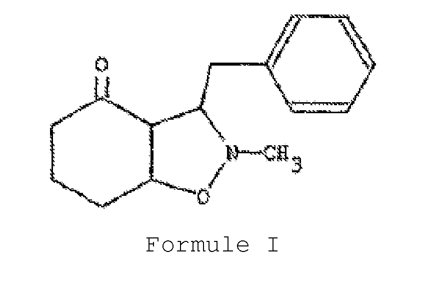 pour un usage en tant que médicament.

11. Sel de fumarate selon la revendication 10, qui est un sel de fumarate du composé de formule I sous la forme d'un ou de plusieurs composés stéréochimiques et de leurs mélanges, pour un usage en tant que médicament.

12. Sel de fumarate selon la revendication 11, qui est un sel de fumarate du composé de formule I sous la forme d'un diastéréoisomère ou d'un mélange racémique, pour un usage en tant que médicament.

13. Sel de fumarate selon l'une quelconque des revendications 10 à 12, qui est le fumarate de rel-(3R,3aS,7aS)-3-benzyl-2-méthyl-2,3,3a,4,5,6,7,7a-octahydrobenzo[d]isoxazol-4-one, pour un usage en tant que médicament.

14. Composition pharmaceutique contenant le sel de fumarate selon l'une quelconque des revendications 1 à 4 et un support pharmaceutiquement acceptable.

15. Composition selon la revendication 14, dans laquelle le sel de fumarate est présent en une quantité de 0,1 mg à 100 mg.

16. Comprimé contenant le sel de fumarate selon l'une quelconque des revendications 1 à 4 et des excipients appropriées.

17. Comprimé selon la revendication 16, dans lequel les excipients appropriés sont choisis dans le groupe constitué par l'amidon pré-gélatinisé, la cellulose microcristalline, le glycolate d'amidon sodique, le talc, le lactose, le stéarate de magnésium, le saccharose, l'acide stéarique et le mannitol.

18. Comprimé selon la revendication 16 ou la revendication 17, dans lequel le sel de fumarate est présent en une quantité allant de 1 mg à 100 mg par comprimé unitaire.

19. Comprimé selon la revendication 18, dans lequel le sel de fumarate est présent en une quantité allant de 1 mg à 50 mg par comprimé unitaire.

20. Utilisation du sel de fumarate du composé de formule I : 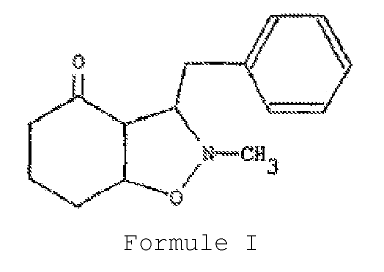 pour la préparation d'un médicament destiné au traitement des troubles de l'humeur, des troubles de l'anxiété, de la dépression et des états convulsifs, à améliorer la capacité d'apprentissage, à inverser une amnésie, à résoudre le syndrome de sevrage liés aux drogues et à la toxicomanie.

21. Utilisation selon la revendication 20, dans laquelle le sel de fumarate est un fumarate du composé de formule I sous la forme d'un ou de plusieurs composés stéréochimiques et de leurs mélanges, pour la préparation d'un médicament destiné au traitement des troubles de l'humeur, des troubles de l'anxiété, de la dépression et des états convulsifs, à améliorer la capacité d'apprentissage, à inverser une amnésie, à résoudre le syndrome de sevrage liés aux drogues et à la toxicomanie.

22. Utilisation selon la revendication 21, dans laquelle le sel de fumarate est un fumarate du composé de formule I sous la forme d'un diastéréoisomère ou d'un mélange racémique, pour la préparation d'un médicament destiné au traitement des troubles de l'humeur, des troubles de l'anxiété, de la dépression et des états convulsifs, à améliorer la capacité d'apprentissage, à inverser une amnésie, à résoudre le syndrome de sevrage liés aux drogues et à la toxicomanie.

23. Utilisation selon l'une quelconque des revendications 20 à 22, dans laquelle le sel de fumarate est le fumarate de rel-(3R,3aS,7aS)-3-benzyl-2-méthyl-2,3,3a,4,5,6,7,7a-octahydrobenzo[d]isoxazol-4-one, pour la préparation d'un médicament destiné au traitement des troubles de l'humeur, des troubles de l'anxiété, de la dépression et des états convulsifs, à améliorer la capacité d'apprentissage, à inverser une amnésie, à résoudre le syndrome de sevrage liés aux drogues et à la toxicomanie.

24. Utilisation selon l'une quelconque des revendications 21 à 23, dans laquelle le médicament est pour une administration par voie orale.

25. Utilisation selon la revendication 24, dans laquelle le médicament pour administration par voie orale est choisi dans le groupe constitué par les comprimés, les capsules, les cachets, les poudres, les granulés ou les suspensions dans un liquide.

26. Utilisation selon la revendication 24 ou la revendication 25, dans laquelle le sel de fumarate du composé de formule I est en une quantité allant de 1 mg à 100 mg par unité de médicament.

27. Utilisation selon la revendication 26, dans laquelle le médicament est un comprimé, où le sel de fumarate du composé de formule I est en une quantité allant de 1 mg à 100 mg par comprimé unitaire.

28. Utilisation selon l'une quelconque des revendications 20 à 23, dans laquelle le médicament est pour une administration par voie topique.

29. Utilisation selon la revendication 28, dans laquelle le médicament pour administration par voie topique est choisi dans le groupe constitué par les crèmes, les huiles, les pommades, les émulsions, les gels, les solutions aqueuses, les solutions à pulvériser et les emplâtres.

30. Utilisation selon la revendication 28 ou la revendication 29, dans laquelle le sel de fumarate du composé de formule I est en une quantité allant de 1 mg à 100 mg par unité de médicament.

31. Utilisation selon l'une quelconque des revendications 20 à 23, dans laquelle le médicament est pour une administration par voie parentérale.

32. Utilisation selon la revendication 31, dans laquelle le médicament pour administration par voie parentérale est une préparation aqueuse stérile.

33. Utilisation selon la revendication 31 ou la revendication 32, dans laquelle le sel de fumarate du composé de formule I est en une quantité allant de 0,1 mg à 100 mg par unité de médicament.

34. Sel de fumarate du composé de formule I : 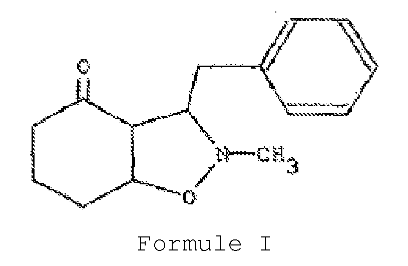 utilisable pour traiter les troubles de l'humeur, les troubles de l'anxiété, la dépression et les états convulsifs, pour améliorer la capacité d'apprentissage, pour inverser une amnésie, pour résoudre le syndrome de sevrage liés aux drogues et à la toxicomanie.
